# EUROPÄISCHE PATENTSCHRIFT

(11) **EP 3 823 464 B1**
(45) Veröffentlichungstag und Bekanntmachung des Hinweises auf die Patenterteilung: **14.08.2024**
(21) Anmeldenummer: 19734777.6
(22) Anmeldetag: 28.06.2019
(51) Int. Cl.: A23L 5/20, A61K 36/185, A61K 36/28, A61K 36/38, A61K 36/53, A23L 33/105, A61K 8/97, A61K 8/9789, A61Q 19/00

(54) **VERFAHREN ZUR ENTFERNUNG VON VERUNREINIGUNGEN AUS PFLANZLICHEN ZUBEREITUNGEN**
METHOD FOR REMOVING IMPURITIES FROM VEGETABLE PREPARATIONS
PROCÉDÉ D'ÉLIMINATION D'IMPURETÉS DE PRÉPARATIONS VÉGÉTALES

(30) Priorität: 17.07.2018 CH 8822018
(43) Veröffentlichungstag der Anmeldung: 26.05.2021
(73) Patentinhaber: Frutarom Schweiz AG, 8820 Wädenswil (CH)
(72) Erfinder: KREUTER, Matthias-Heinrich, 8880 Walenstadt (CH); HOFER, Florian, 5000 Aaarau (CH)
(74) Vertreter: E. Blum & Co. AG
(86) Internationale Anmeldenummer: PCT/EP2019/067400
(87) Internationale Veröffentlichungsnummer: WO 2020/015983

(56) Entgegenhaltungen:
- EP-A1- 0 673 654
- EP-A1- 0 730 830
- EP-A1- 3 159 002
- EP-B1- 0 730 830

## Beschreibung

### Gebiet der Erfindung

Die vorliegende Erfindung liegt im technischen Gebiet von pflanzlichen Zubereitungen und betrifft ein Verfahren zur Entfernung von unerwünschten Verunreinigungen aus pflanzlichen Zubereitungen sowie gereinigte pflanzliche Zubereitungen und Verwendungen der gereinigten pflanzlichen Zubereitungen.

### Hintergrund

Pflanzliche Zubereitungen enthalten oft unerwünschte Schadstoffe und Verunreinigungen. Insbesondere betrifft dies die Kontamination durch Pyrrolizidin-Alkaloide sowie auch Kontamination durch unerwünschte lipophile Verunreinigungen, wie zum Beispiel polyzyklische aromatische Kohlenwasserstoffe oder Pflanzenschutzmittel.

Pyrrolizidin-Alkaloide sind eine Gruppe in der Natur häufig vorkommender sekundärer Pflanzeninhaltsstoffe. Einige im menschlichen Körper entstehenden Abbauprodukte von Pyrrolizidin-Alkaloiden wirken in geringen Dosierungen und/oder bei chronischer Exposition stark leberschädigend. Ebenfalls nachweisbar sind eine erbgutverändernde und krebserregende Wirkung.

Durch die häufig vorkommende Kontaminierung des pflanzlichen Ausgangsmaterials für die Herstellung pflanzlicher Zubereitungen mit pyrrolizidin-alkaloidhaltigen Unkräutern, sind auch Produkte aus generell unbedenklichen Pflanzengattungen von dieser Problematik betroffen.

Aus diesem Grund wurde im Jahr 2014 von der HMPC (Committee on Herbal Medicinal Products) eine Empfehlung über die Limitierung der Pyrrolizidin-Alkaloid-Aufnahme auf 0.35 pg pro Tag herausgegeben (Public statement on contamination of herbal medicinal products / traditional herbal medicinal products with pyrrolizidin alkaloids. 31.03.2016 EMEA/HMPC/328782/2016). Am 1. März 2016 wurde in Deutschland für pflanzliche Arzneimittel eine Übergangs-Limite für die tägliche maximale Einnahme von 1 pg Pyrrolizidin-Alkaloide pro Tag festgelegt. Österreich und Grossbritannien haben sich diesem Vorstoss kurze Zeit später angeschlossen.

Pyrrolizidin-Alkaloide und manche andere Verunreinigungen oder Schadstoffe und auch bestimmte lipophile Kontaminanten (beispielsweise Polyaromate) können durch Verwendung von sogenannten Adsorbenzien, beispielsweise Aktivkohle, abgereichert werden. Dies beruht auf der Eigenschaft von Aktivkohle, als unspezifisches Adsorbens Substanzen zu adsorbieren. Ein solches Reinigungsverfahren ist allerdings kostenintensiv, nicht selektiv und adsorbiert ebenfalls andere Pflanzeninhaltsstoffe, unter anderem auch diverse Wirksubstanzen in pflanzlichen Arzneimitteln und ist daher für die Dekontamination pflanzlicher Zubereitungen sowohl in qualitativer als auch quantitativer Hinsicht ungeeignet.

Weissem Ton (Bolus alba), einem Zweischicht-Tonmineral, wird ebenfalls die Eigenschaft zugeschrieben als Adsorbens zu wirken und insbesondere basische Stoffe zu binden, beispielsweise als Antidot zur Bindung von Alkaloiden im Magen-Darmtrakt. Weisser Ton bindet in pflanzlichen Zubereitungen jedoch Pyrrolizidin-Alkaloide nicht.

Selektive Reinigungsverfahren wie in EP 0`730`830 dargestellt, reichern erfolgreich lipophile Verunreinigungen ab, erfassen jedoch Pyrrolizidin-Alkaloide nicht.

EP 3'159'002 beschreibt unter anderem ein Verfahren zur Herstellung eines Spezial-Extrakts aus Symphytum, umfassend die selektive Entfernung von Pyrrolizidin Alkaloiden.

Ferner beschreibt EP 0'673' 654 ein Verfahren zur Herstellung eines Symphytum-Extraktes, bei dem der Pyrrolizidinalkaloidgehalt auf einen gewünschten Wert reduziert wird.

### Darstellung der Erfindung

Aufgabe der vorliegenden Erfindung ist es Pyrrolizidin-Alkaloide aus pflanzlichen Zubereitungen selektiv zu entfernen. Dabei sollen insbesondere oben genannte Nachteile von im Stand der Technik bekannten Verfahren überwunden werden.

In einem ersten Aspekt der Erfindung wird diese Aufgabe gelöst durch ein Verfahren Entfernung von Verunreinigungen umfassend mindestens ein Pyrrolizidin-Alkaloid aus einer pflanzlichen Zubereitung. Das erfindungsgemässe Verfahren zur Entfernung umfasst folgende Schritte:
- Schritt 1: Bereitstellen einer pflanzlichen Zubereitung in flüssiger Form;
- Schritt 2: Kontaktieren der pflanzlichen Zubereitung mit einem Bentonit, um die zu entfernenden Verunreinigungen zu adsorbieren;
- Schritt 3: Abtrennen des Bentonits mit daran gebundenen Verunreinigungen von der pflanzlichen Zubereitung.
Bentonit eignet sich überraschenderweise als mineralisches Adsorbens für eine selektive Entfernung von Pyrolizidin-Alakaloiden aus einer pflanzlichen Zubereitung. Es ist bekannt, dass pflanzliche Zubereitungen neben erwünschten Inhaltsstoffen wie beispielsweise pharmazeutische Wirkstoffe, wie Flavonoide, Vitamine, Duftstoffe oder Aromastoffe auch schädliche Pyrrolizidin-Alkaloide oder auch andere, beispielsweise fettlösliche Schadstoffe enthalten. Es ist ein grosser Vorteil des erfindungsgemässen Reinigungsverfahrens, dass es den Gehalt an Pyrrolizidin-Alkaloiden durch selektive Entfernung reduziert, während der Gehalt an erwünschten Inhaltsstoffen wie beispielsweise Flavonoide nicht oder nur unwesentlich reduziert wird.

Der Begriff Schadstoff umfasst insbesondere Verunreinigungen und Schadstoffrückstände in pflanzlichen Zubereitungen. Verunreinigungen umfassen Stoffe, welche unabsichtlich und unerwünscht in den pflanzlichen Zubereitungen vorhanden sind. Sie stammen beispielsweise von sogenannten Beikräutern oder Fremdpflanzen in der Pflanzenernte, welche Toxine wie Alkaloide oder auch Stoffe mit unerwünschter pharmazeutischer Aktivität enthalten. Manche Pflanzen, enthalten abgesehen von den erwünschten Wirkstoffen jedoch auch selber unerwünschte Schadstoffe oder Gifte, welche zusammen mit den erwünschten Wirkstoffen durch Extraktion in pflanzliche Zubereitungen gelangen. Andere beispielhafte Verunreinigungen umfassen Mykotoxine, insbesondere Ochratoxine, verursacht durch Schimmelpilzbefall der Pflanzen auf dem Feld. Schadstoffrückstände in pflanzlichen Zubereitungen umfassen insbesondere Rückstände von Pflanzenschutzmitteln und Schädlingsbekämpfungsmitteln und umfassen auch lipophile Schadstoffe und Rückstände.

Für das erfindungsgemässe Verfahren wird eine pflanzliche Zubereitung in flüssiger Form bereitgestellt. In diesem Text umfasst der Begriff pflanzliche Zubereitung einen Extrakt enthaltend pflanzliche Inhaltsstoffe. Erwünschte Inhaltsstoffe der pflanzlichen Zubereitung umfassen erwünschte Wirk- und Leitstoffe. Unerwünschte Inhaltsstoffe der pflanzlichen Zubereitung umfassen Schadstoffe, insbesondere Verunreinigungen wie insbesondere Pyrrolizidin-Alkaloide. Zielpflanzen zur Herstellung der bereitgestellten pflanzlichen Zubereitung sind insbesondere Extrakte aus Heil-, Gewürzpflanzen und/oder Aromapflanzen oder Teilen davon.

Geeignete pflanzliche Zubereitungen zur Behandlung mit dem erfindungsgemässen Verfahren sind insbesondere Aufgüsse, Tinkturen, Fluida, Spissum-Extrakte, Tropflösungen, Decocte, Mazerate, Digestione und Tonika oder flüssige Zwischenprodukte getrockneter Zubereitungen oder flüssige Zubereitungen, die aus Konzentraten oder aus getrockneten Zubereitungen hergestellt werden.

Der Begriff mindestens ein Pyrrolizidin-Alkaloid umfasst (ein oder mehrere) Alkaloide, deren Grundstruktur das Ringsystem Pyrrolizidin enthält. Der Begriff Pyrrolizidin-Alkaloid umfasst auch Untergruppen wie beispielsweise Pyrrolizidin-Alkaloide des Senecionin-Typs, insbesondere 1,2 ungesättigte Necine, inbes. Retronecin, oder Pyrrolizidin-Alkaloide des Lycopsamin-Typs oder des Triangularin-Typs sowie deren Aminoxide (N-Oxide).

Das erfindungsgemässe Verfahren beruht auf einer Behandlung der flüssigen pflanzlichen Zubereitung mit dem mineralischen Adsorbens Bentonit.

Als Bentonit wird in diesem Text ein Tonmineral bezeichnet das mindestens 50% Smektit enthält. Smektit ist ein Dreischicht-Silikat und umfasst quellfähige Tonminerale der dioktaedrischen Montmorillonit-Reihe sowie der trioktaedrischen Saponit-Reihe (s. Zusammensetzung gemäss der Strunz Systematik). Der für das Verfahren verwendete Bentonit besteht insbesondere zu einem grossen Teil, beispielsweise zu 60-80%, aus Montmorillonit ((Na,Ca)_{0,3}(Al,Mg)₂Si₄O₁₀(OH)₂ x nH₂O).

Bentonit findet dank seiner Fähigkeit zu grosser Wasseraufnahme und starken Quellfähigkeit in vielen Bereichen der Industrie und Bautechnik, Lebensmitteltechnologie, Pharmazie und Kosmetik Anwendung. Als pharmazeutischer Hilfsstoff dient Bentonit beispielsweise als Stabilisator, Füll- oder Verdickungsmittel. In der Getränketechnologie dient Bentonit beispielsweise als mineralisches Adsorbens, zur Klärung von Eiweiss- und anderen Kolloidaltrübungen. Kommerziell erhältliche und zu diesem Zweck behördlich zugelassene Produkte sind z.B. NaCalit PORE-TEC & Blancobent UF (ERBSLÖH). In manchen Ausführungsformen des erfindungsgemässen Verfahrens werden Bentonit Produkte verwendet, welche für Anwendungen der Getränke- oder Lebensmitteltechnologie oder für die Herstellung oder als Beistoff pharmazeutischer Produkte zugelassen sind. In manchen Ausführungsformen wird Bentonit chemisch entsprechend oder insbesondere gemäss der Definition der europäischen Pharmakopöe, Ph. Eur., 01/2017:0467 verwendet.

Es ist ein grosser Vorteil der Erfindung, dass Bentonit Pyrrolizidin-Alkaloide in flüssigen Pflanzenzubereitungen selektiv adsorbiert. Das heisst Pyrrolizidin-Alkaloide werden in dem Sinne selektiv entfernt, als dass erwünschte Wirk- und Leitstoffe nicht oder nur in einem unwesentlichen Mass auch an Bentonit adsorbieren und entfernt werden.

In manchen Ausführungsformen sind die erwünschten Inhaltsstoffe bzw. Wirkstoffe ausgewählt aus der Gruppe umfassend Polyphenole, insbesondere Rosmarinsäure oder Flavonoide und Flavone wie Rutin, Apigenin oder Vitexin, Hypericine, wie Hypericin, Saponine, Isoprenoide und Iridoide, ätherische Öle, Alkylamide, Pflanzensäuren, wie zum Beispiel, Cichoriensäure, Lignane, Anthocyane, Anthracen-Derivate, Terpene, beispielsweise Sesquiterpenlactone oder Polyterpene, Cumarine, Glukosinolate, Bitterstoffe und Allantoin. In manchen Ausführungsformen sind die erwünschten Inhaltsstoffe bzw. Wirkstoffe ausgewählt insbesondere aus ätherische Öle, insbesondere aus Flavonoide und Flavone, insbesondere aus Polyphenole, insbesondere aus Saponine und / oder insbesondere aus Sesquiterpenlactone und Polyterpene.

Im Gegensatz zu Bentonit bewirken viele andere im Stand der Technik bekannte mineralische Adsorbenzien keine solche selektive Adsorbtion. Beispielsweise sind Kieselgur (Diatomit, amorphes Siliziumdioxid), Magnesiumsilikate wie z.B. Magnesiumtrisilikat (2 MgO x 3 SiO₂ x n H₂O; Ph.Eur)oder Magnesol (2 MgO x 3 SiO₂ x n H₂O; Ph.Eur), Tricalziumphosphat, Talk (Mg₃[Si₄O₁₀(OH)₂]), Calciumhydrogenphosphat oder Kaolin (Bolus Alba / Weisser Ton; Al₂O₃ x 2 SiO₂ x 2 H₂O) und Kaolinit (Al₄[(OH)₈|Si₄O₁₀] nicht geeignet zur selektiven Adsorption von Pyrrolizidin-Alkaloiden.

Ein weiterer Aspekt der Erfindung betrifft die Verwendung von Bentonit zur Entfernung von Pyrrolizidin-Alkaloiden aus pflanzlichen Zubereitungen oder aus flüssigen Zwischenprodukten von getrockneten Zubereitungen.

Die in diesem Text im Zusammenhang mit dem Verfahren zur Entfernung von Pyrrolizidin-Alkaloiden definierten Begriffe gelten auch im Zusammenhang mit der Verwendung von Bentonit zur Entfernung von Pyrrolizidin-Alkaloiden aus pflanzlichen Zubereitungen sowie weiteren beschriebenen Aspekten der Erfindung. Die weiteren Aspekte der Erfindung umfassen die Verwendung von Bentonit zur Reinigung von pflanzlichen Zubereitungen, Herstellungsverfahren zur Herstellung gereinigter pflanzlicher Zubereitungen, sowie gereinigte pflanzliche Zubereitungen erhältlich durch ein Verfahren umfassend die Verwendung von Bentonit und insbesondere gereinigte pflanzliche Zubereitungen zur therapeutischen Verwendung und weitere Verwendungen gereinigter pflanzlichen Zubereitungen, beispielsweise in Kosmetika oder in Lebensmittel oder als Nahrungsergänzungsmittel.

Ferner betreffen die in diesem Text beschriebenen und in den abhängigen Ansprüchen beanspruchten bevorzugten Ausführungsformen des Verfahrens insbesondere auch bevorzugte Ausführungsformen der Verwendung von Bentonit, bzw. deren Produkte.

Der Gehalt an Pyrrolizidin-Alkaloiden in der pflanzlichen Zubereitung vor der Behandlung mit Bentonit kann sehr stark variieren. Der Gehalt ist beispielsweise abhängig von der extrahierten Pflanzenart und kann selbst in Extrakten der gleichen Pflanzenart aus verschiedenen Pflanzenlieferungen stark variieren. Es ist ein grosser Vorteil, dass durch die Behandlung der flüssigen pflanzlichen Zubereitung mit Bentonit ein reproduzierbar niedriger Gehalt an Pyrrolizidin-Alkaloiden in der gereinigten Zubereitung gewährleistet wird. Dadurch kann die Einhaltung von Grenzwerten für den Pyrrolizidin-Alkaloid Gehalt in gereinigten pflanzlichen Zubereitungen gemäss Vorschriften der zuständigen Behörden gewährleistet werden. Je nach Gehalt im Ausgangsmaterial und je nach Höhe des Grenzwertes kann dazu eine nur geringe Reduktion des Gehalts bereits ausreichen oder aber es ist eine grössere Abreicherung notwendig, um einen Grenzwert zu erfüllen.

Beispielsweise genügt in manchen Ausführungsformen des Verfahrens eine Abreicherung von mindestens einem Pyrrolizidin-Alkaloid um einen Faktor 1.1, entsprechend einer Reduktion des Gehalts an Pyrrolizidin-Alkaloiden in der gereinigten Zubereitung um 9% bezogen auf den Gehalt an Pyrrolizidin-Alkaloiden in der unbehandelten Zubereitung zur Einhaltung eines bestimmten Grenzwertes. Es kann dazu auch eine grössere Abreicherung notwendig sein, beispielsweise um einen Faktor von mindestens 1.25, 1.5, 2, 3, 4, 5 oder 10 entsprechend einer Reduktion des Gehalts in der gereinigten Zubereitung um mindestens 20%, 33%, 50%, 67%, 80% oder 90% bezogen auf den Gehalt in der unbehandelten Zubereitung.

Manche Anwendungen des erfindungsgemässen Verfahrens und der erfindungsgemässen Verwendung von Bentonit betreffen eine Behandlung von pflanzlichen Zubereitungen, in welchen eine Belastung mit einem Pyrrolizidin-Alkaloid selten vorkommt oder sehr gering ist und möglicherweise an der Grenze oder unter einem Grenzwert oder unterhalb einer Messschwelle liegt. Auch solche Anwendungen des Verfahrens sind geeignet, um insbesondere für die industrielle Produktion die routinemässige Einhaltung von Grenzwerten für Pyrrolizidin-Alkaloide in der gereinigten Zubereitung zu gewährleisten. In manchen Ausführungsformen des erfindungsgemässen Verfahrens wird eine nahezu quantitative Entfernung erzielt.

Die behördlich festgelegten Grenzwerte variieren beispielsweise in Abhängigkeit von der massgeblichen zuständigen Behörde, des Pyrrolizidin-Alkaloids, der pflanzlichen Zubereitung und deren Verwendungszweck. Insbesondere ist die massgebliche zuständige Behörde ausgewählt aus Behörden der Vereinigten Staaten Amerikas, der Europäischen Union, Israels und der Schweiz, insbesondere die U.S. Food and Drug Administration FDA und die European Medicines Agency EMA. In manchen Ausführungsformen des Verfahrens wird ein Grenzwert eines maximalen Gehalts von mindestens einem Pyrrolizidin-Alkaloid, eingehalten, wobei der Grenzwert ein durch die FDA oder EMA festgelegter Grenzwert in der gereinigten pflanzlichen Zubereitung ist. Dabei gilt die jeweils aktuellste Bestimmung dieser Behörden bzw. der aktuellste massgebliche Grenzwert oder insbesondere der im Jahre 2018 massgebliche Grenzwert wie beispielsweise gemäss der U.S. Food & Drug Administration, Environmental Protection Agency Pesticide Tolerances Guideline 11/06/2017 oder E-CFR Part 180 Tolearances and Exemptions for Pesticide Chemical in Food, April 6, 2018.

Es ist ein grosser Vorteil der Erfindung, dass nationale und internationale Vorgaben zu Höchstmengen von Pyrrolizidin-Alkaloiden zuverlässig eingehalten oder sogar deutlich unterschritten werden können, ohne dass dadurch der Gehalt an erwünschten Wirkstoffen und analytischen Markern (Leitstoffe) wesentlich verändert wird. Der Gehalt an erwünschten Wirk- und Leitstoffen, wie beispielsweise Polyphenole, insbesondere Rosmarinsäure oder Flavonoide und Flavone wie Rutin, Apigenin oder Vitexin, Hypericine, wie Hypericin, Saponine, Isoprenoide und Iridoide, ätherische Öle, Alkylamide, Pflanzensäuren, wie zum Beispiel, Cichoriensäure, Lignane, Anthocyane, Anthracen-Derivate, Terpene, beispielsweise Sesquiterpenlactone oder Polyterpene, Cumarine, Glukosinolate, Bitterstoffe, Allantoin und andere wird in Anwendungen des Verfahrens um weniger als 30%, insbesondere um weniger als 20% oder weniger als 10% oder weniger als 5% reduziert.

Somit hat das Verfahren keinen Einfluss auf das qualitative und quantitative Spektrum erwünschter Pflanzeninhaltsstoffe, der über die naturgegebenen Schwankungen hinausgeht. Dies ist ein grosser Vorteil, denn ein Verlust charakteristischer erwünschter Inhaltsstoffe in pflanzlichen Zubereitungen würde bedeuten, dass deren erwiesene Wirksamkeit und Unbedenklichkeit nicht mehr gewährleistet wäre. Weitere Vorteile des erfindungsgemässen Verfahrens zur Entfernung von Pyrrolizidin-Alkaloiden sind, dass es ein kostengünstiges, umweltschonendes und einfach anwendbares Verfahren ist. Ferner werden vorteilhafterweise keine toxische oder leicht entflammbare Mittel in dem Verfahren verwendet.

Ein weiterer Aspekt der Erfindung betrifft ein Herstellungsverfahren einer gereinigten pflanzlichen Zubereitung, welches die Verwendung von Bentonit zur Entfernung von mindestens einem Pyrrolizidin-Alkaloid umfasst, wodurch eine gereinigte pflanzliche Zubereitung erhalten wird.

### Wege zur Ausführung der Erfindung

Es versteht sich, dass die verschiedenen in diesem Text offenbarten und beschriebenen Ausführungsformen der Erfindung beliebig miteinander kombiniert werden können. Ausserdem können in gewissen Ausführungsformen einzelne Definitionen, Bevorzugungen und Bereiche nicht zur Anwendung kommen.

Vorteilhafterweise lässt sich die zugegebene Menge von Bentonit zur pflanzlichen Zubereitung insbesondere im Schritt 2 des Verfahrens je nach Anwendung variieren. Bentonit kann insbesondere in einer Menge von 5 bis 200% m/m bezogen auf den Trockensubstanzgehalt der Zubereitung zugegeben werden. Die Menge an zugegeben Bentonit kann variieren beispielsweise je nach der Pflanzenart und -gattung aus welcher die Zubereitung extrahiert wurde, je nach dem Pyrrolizidin-Alkaloidgehalt in der unbehandelten pflanzlichen Zubereitung oder insbesondere je nach dem notwendigen Grad der Abreicherung. Beispielsweise kann die zu verwendende Menge Bentonit für die Einhaltung eines durch einen Grenzwert vorgegebenen maximal tolerierbaren Pyrrolizidin-Alkaloidgehalt in der gereinigten Zubereitung angepasst werden.

Eine Erhöhung der zugegebenen Menge an Bentonit bewirkt eine stärkere Abreicherung des Pyrrolizidin-Alkaloidgehalts in der pflanzlichen Zubereitung, d.h. eine Erhöhung des Faktors, um welchen der Gehalt in der gereinigten Zubereitung im Vergleich zur unbehandelten pflanzlichen Zubereitung reduziert ist. Diese positive Wirkung von ansteigender Menge an zugegebenem Bentonit ist beispielsweise aus dem Vergleich der Resultate von Beispiel 1 und 3 (Tabelle 1), der Beispielreihe 5 bis 7 (Tabelle 2), Beispielreihe 10, 11, 13, 14 (Tabelle 3)oder der Beispielreihe 16 bis 18 (Tabelle 5) ersichtlich. Bentonit wird beispielsweise in einer Mindestmenge von Masse Bentonit zu Trockensubstanzmasse in der bereitgestellten pflanzlichen Zubereitung vor der Behandlung von mindestens 10% m/m, 20% m/m, 40% m/m, 50% m/m, 70% m/m oder 100% m/m zugegeben. Erfahrungsgemäss insbesondere auch gemäss den Beispielen bewirkt eine Verwendung von 5 bis 10% m/m Bentonit bereits eine Abreicherung des Schadstoffgehalts um mindestens einen Faktor 1.5 und meistens über einen Faktor 2. Vorteilhafterweise bleibt auch bei Zugabe von hohen Mengen an Bentonit zur Erhöhung des Abreicherungsfaktors die Selektivität der Entfernung von Pyrrolizidin-Alkaloiden erhalten. Das heisst während der Schadstoffgehalt in der gereinigten pflanzlichen Zubereitung bei zunehmender Menge an verwendetem Bentonit abnimmt, wird der Gehalt an erwünschten Inhaltsstoffen nicht oder nicht wesentlich reduziert. Dies geht ebenfalls aus den Resultaten oben genannter Beispiele betreffend erwünschte Leit- und Wirkstoffe hervor (s. Tabellen 1, 3 und 4). Dennoch ist es aus praktischen Gründen (z.B. Volumengrösse, Dauer des Filtrationsverfahrens, Kosten) vorteilhaft, nicht eine unnötig grosse Menge an Bentonit zu verwenden, sondern diese zu beschränken insbesondere auf bis zu 30% m/m, 40% m/m, 50% m/m, 70 % m/m oder 100% m/m Bentonit bezogen auf den Trockensubstanzgehalt der Zubereitung.

In manchen Ausführungsformen des Verfahrens ist das mindestens eine Pyrrolizidin-Alkaloid ausgewählt aus der Gruppe umfassend
- Pyrrolizidin-Alkaloide des Senecionin-Typs sowie deren Aminoxide, insbesondere 1,2 ungesättigte Necine, inbesondere Retronecin, sowie deren Aminoxide,
- Pyrrolizidin-Alkaloide des Lycopsamin-Typs sowie deren Aminoxide, und
- Pyrrolizidin-Alkaloide des Triangularin-Typs sowie deren Aminoxide.

Manche Ausführungsformen des Verfahrens umfassen weitere Schritte, die vor, zwischen oder nach den Schritten 1 bis 3 durchgeführt werden.

Zusätzliche Verfahrensschritte dienen beispielsweise der zusätzlichen Entfernung von mindestens einem lipophilen Schadstoff. Dazu wird in einem Schritt A die pflanzliche Zubereitung mit einer geeigneten lipophilen Phase in Verbindung gebracht, um die zu entfernenden Schadstoffe in dieser lipophilen Phase zu lösen und anzureichern. In einem Schritt B wird die lipophile Phase mit den darin enthaltenen Schadstoffen von der pflanzlichen Zubereitung getrennt. Die Schritte zur Entfernung von lipophilen Schadstoffen können insbesondere vor oder nach den Schritten zur Entfernung von mindestens einem Pyrrolizidin-Alkaloid durchgeführt werden. Die beiden Schritte A und B werden also vor dem zweiten oder nach dem dritten Schritt des Verfahrens durchgeführt. Alternativ können zuerst die Schritte 2 und A und danach die Schritte 3 und B durchgeführt werden. Dabei ist Schritt A vor, nach oder gleichzeitig mit dem zweiten Schritt und Schritt B vor, nach oder gleichzeitig mit dem dritten Schritt durchführbar. Ein geeignetes Verfahren zur Entfernung von lipophilen Schadstoffen ist in EP0730830 beschrieben. Das hier beschriebene Verfahren zur Entfernung von Verunreinigungen umfassend mindestens ein Pyrrolizidin-Alkaloid kann beliebig mit den in EP 0 730 830 B1 dargestellten Schritten ergänzt und/oder kombiniert werden um auch unerwünschte lipophile Verunreinigungen zu entfernen.

Der zu entfernende lipophile Schadstoff wird insbesondere ausgewählt aus der Gruppe von organischen Verbindungen umfassend polyaromatische Kohlenwasserstoffe, insbesondere Benzo[a]anthracen, Chrysen, Benzo[b]fluoran-then, Benzo[a]pyren; polyhalogenierte, insbesondere polychlorierte Biphenyle, Alkylhalogenide und halogenierte Aromate und halogenierte Heteroaromate, insbesondere Chlorpyridine.

Als lipophile Schadstoffe werden in diesem Text Schadstoffe bezeichnet, welche löslich oder nahezu löslich, insbesondere mindestens zu 98% löslich sind in Lipiden oder Lipoiden (Lipid-ähnlichen Stoffen). Die genannten organischen Verbindungen sind dem Fachmann bekannt und insbesondere im Römpp Lexikon Chemie verzeichnet.

In manchen Ausführungsformen der Erfindung werden zur Herstellung der bereitgestellten pflanzlichen Zubereitung Pflanzen verwendet, welche die erwünschten Inhaltsstoffe enthalten und selber keine Pyrrolizidin-Alkaloide führen. Pyrrolizidin-Alkaloide in pflanzlichen Zubereitungen, welche aus nicht-Pyrrolizidin-Alkaloid-führenden Pflanzen stammen, gelangen als Verunreinigung während der Herstellung durch Pyrrolizidin-Alkaloide-führende Fremdpflanzen wie Bei- oder Unkräuter in die bereitgestellte pflanzliche Zubereitung.

In manchen Ausführungsformen des Verfahrens werden zur Herstellung der bereitgestellten pflanzlichen Zubereitung Pyrrolizidin-Alkaloid führende Pflanzen verwendet. In diesen Ausführungsformen stammt die zu entfernende Pyrrolizidinalkaloid Verunreinigung nicht oder nicht nur aus Fremdpflanzen, sondern aus oder auch aus der Zielpflanze für die Herstellung der bereitgestellten pflanzlichen Zubereitung, welche als Inhaltsstoffe nicht nur erwünschte Inhaltsstoffe, sondern auch mindestens ein Pyrrolizidin-Alkaloid enthält. Insbesondere ist der mindestens eine erwünschte Inhaltsstoff einer Pyrollizidin-Alkaloid führenden Pflanze ausgewählt aus der Gruppe umfassend Polyphenole, insbesondere Rosmarinsäure oder Flavonoide und Flavone wie Rutin, Apigenin oder Vitexin, Hypericine, wie Hypericin, Saponine, Isoprenoide und Iridoide, ätherische Öle, Alkylamide, Pflanzensäuren, wie zum Beispiel, Cichoriensäure, Lignane, Anthocyane, Anthracen-Derivate, Terpene, beispielsweise Sesquiterpenlactone oder Polyterpene, Cumarine, Glukosinolate, Bitterstoffe und Allantoin.

Pyrrolizidin-Alkaloid führende Pflanzen und deren Pflanzenteile sind beispielsweise ausgewählt aus der Gruppe umfassend
- Borago, insbesondere Folia
- Eupatorium, insbesondere Herba
- Pulmonaria, insesondere Herba
- Senecio, insbesondere Herba
- Symphytum, insbesondere Herba
- Tussilago, insbesondere Herba

In manchen Ausführungsformen des Verfahrens wird eine pflanzliche Zubereitung bereitgestellt, welche insbesondere hergestellt ist aus der Gruppe der nachfolgend aufgelisteten Zielpflanzen, wobei bevorzugte Pflanzenteile in Klammern genannt sind:
- Abelmoschus moschatus L (Semen)
- Acorus calamus (Rhizom)
- Aesculus hippocastanum L. (Semen)
- Alchemilla sp. L. (Herba)
- Allium-Arten (z.B. A. cepa L., A. ursinum L., A. sativum L.:Bulbus)
- Aloysia citrodora Palau (Herba)
- Alpinia officinarum Hance (Rhizom)
- Althaea officinalis L. (Radix)
- Anethum graveolens L. (Fructus)
- Angelica archangelica L., verschiedene Subspec. (Rhizoma)
- Angelica dahurica (Radix)
- Angelica formosana (Radix)
- Anthemis nobilis L. ( Flores)
- Apium graveolens L. (Fructus)
- Arctium major Gaertn. (Radix)
- Arctostaphylos uva-ursi Spreng. (Folium)
- Arnica montana L. (Flos)
- Artemisia absinthium L. (Herba)
- Artemisia dracunculus L. (Herba)
- Aspalathus linearis R.Dahlgren (Folium)
- Asparagus offic. (Herba, Rhizoma, Radix)
- Avena sativa L. (Herba)
- Betula-Arten (Folium)
- Borago, insbesondere Folia
- Brassica nigra (L.) Koch (Semen)
- Camellia sinensis (Folium)
- Cannabis sativa L. Summitates, Herba)
- Cannabis sativa L. var.indica (Summitates, Herba)
- Capsella bursa-pastoris (Herba)
- Carum carvi L. (Fructus)
- Cetraria islandica (L.) Ach.
- Chinchonae, insbesondere Cortex
- Chrysanthemum vulgare Asch. (Herba)
- Cinnamomum-Arten, (Cortex)
- Citrus-Arten (Folium, Flavedo, Fructus)
- Copaifera reticulata Ducke (Balsam)
- Coriandrum sativum L. (Fructus)
- Crataegus, insbesondere Folia c. Flores
- Cucurbita pepo L. (Semen)
- Cuminum cyminum L. (Fructus)
- Curcuma-Arten (Rhizoma)
- Cusparia officinalis (Willd.) Eng. (Cortex)
- Cynara scolymus L. (Folium)
- Dipterocarpus turbinatus Gaertn. (Balsamum)
- Drosera Arten (D. rotundifolia L., D.ramentacea Burch; Herba)
- Echinacea angustifolia D.C. (Radix)
- Echinacea pallida Nutt. (Radix)
- Echinacea purpurea (L.) Moench (Radix)
- Elettaria cardamonum (L.) White et Mathon (Fructus)
- Eleutherococcus senticosus Maxim. (Radix)
- Equisetum arvense L. (Herba)
- Eucalyptus globulus Labill. (Folium)
- Eupatorium, insbesondere Herba
- Euphrasia sp. L. (Herba)
- Fagopyrum vulgare Hill. (Herba)
- Foeniculum vulgare Miller (Fructus)
- Fumaria offic. (Herba)
- Gaultheria procumbens L. (Folium)
- Ginkgo biloba L. (Folium)
- Ginseng, insbesondere Radix
- Glycine max Merr. (Herba)
- Hamamelis virginiana L. (Cortex, Folium)
- Harpagophytum procumbens DC. (Radix)
- Hedeoma pulegioides (L.) Pers. (Herba)
- Hedera helix L. (Folium)
- Herniaria glabra L. (Herba)
- Hippocastanus, insbesondere Semen
- Humulus lupulus L. (Flos, Glandulae)
- Hypericum perforatum L. (Herba)
- Hysopus officinalis L. (Herba)
- Ilex paraguariensis St. Hil. (Folium mate)
- Illicium verum Hook. f. (Fructus)
- Iluna helenium L. (Rhizoma)
- Iris pallida Lam. (Rhizoma)
- Jasminum grandiflorum L. (Flos)
- Juniperus sp. L. (Fructus)
- Krameria lappacea L. (Radix)
- Laurus nobilis L. (Folium, Fructus)
- Lavendula officinalis, weitere Arten (Flos)
- Lawsonia inermis L. (Folium)
- Levisticum officinale Koch (Radix)
- Linum usitatissimum L. (Fructus)
- Liquiritia, insbesondere Radix
- Malva sylvestris L. (Folium)
- Melaleuca: div. Varietäten (Folium)
- Matricaria recutita L. (Flos)
- Melilotus officinalis (L.) Lam. em. Thuill. (Herba)
- Melissae officinalis L. (Folium)
- Mentha-Arten und ihre Varietäten (Folium)
- Myristica fragrans Houttuyn (Arillus, Semen)
- Myrtus communis L. (Folium)
- Ocimum basilicum L. (Herba)
- Ocotea sassafras (Cortex)
- Oenanthe aquatica (L.) Poir (Fructus)
- Olea europaea (Folium)
- Olibanum (Resinum)
- Ononis spinosa L. (Radix)
- Origanum-Arten (Herba)
- Orthosiphon stamineus Benth. (Herba)
- Panax ginseng Meyer (Radix)
- Passiflora incarnata L. (Herba)
- Pelargonium sidoides DC. (Radix)
- Petroselinum crispum (Mill.) Nym. (Fructus, Herba)
- Phaseolus vulgaris L. (Fructus sine Semine)
- Pimenta dioica (L.) Merill (Fructus)
- Pimpinella anisum L. (Semen)
- Piper angustifolium Ruiz. et Pavon. (Folium)
- Piper methysticum Forster (Radix)
- Pogostemon patchouli Pell. (Folium)
- Portulaca oleracea L.(Herba)
- Primula veris L. (Radix)
- Prunus laurocerasus L. (Folium)
- Pulmonaria, insesondere Herba
- Rhus aromatica Ait. (Cortex)
- Rosmarinus officinalis L. und ihre Species (Folium)
- Rubus fructicosus L. (Folium)
- Ruta graveolens L. (Herba)
- Sabale serulata Benth et Hook (Fructus)
- Salix alba L. (Cortex) und alle Arten
- Salvia-Arten (Folium)
- Santalum album L. (Lignum)
- Sarothamnus scoparius (L.) Wimmer (Herba)
- Sassafras albidum (Nutt.) Nees (Lignum)
- Satureja hortensis L. (Herba)
- Senna acutifolia L. (Folium)
- Schisandra chinensis Baill. (Fructus)
- Solanum betaceum Cav. (Fructus)
- Solidago serotina Ait. (Herba)
- Solidago virgaurea L. (Herba)
- Symphytum, insbesondere Herba
- Syzygium aromaticum Merr. et Perry (Flores, Folium)
- Taraxacum officinale Web. (Herba und Radix)
- Thymus serpyllum L. (Herba)
- Thymus vulgaris L. Herba
- Tilia cordata Mill. und T. platyphyllos Scop. (Flos)
- Trigonella foenum-graecum L. (Herba)
- Tussilago, insbesondere Herba
- Urtica dioica L. (Folium, Radix)
- Valeriana officinalis L.s.I. und ihre Varietäten (Radix)
- Vitex agnus-castus L. (Fructus)
- Vitis vinifera L. (Folium)
- Zingiberis officinale Roscoe (Rhizoma).

Eine bezeichnete Pflanzengattung umfasst in diesem Text, wenn nicht näher definiert, die entsprechenden Arten und eine bezeichnete Pflanzenart umfasst, wenn nicht näher definiert die entsprechenden Varietäten.

In manchen Ausführungsformen des Verfahrens wird eine pflanzliche Zubereitung bereitgestellt, welche besonders häufig von Pyrrolizidin Verunreinigungen betroffen ist - durch die Zielpflanze der Herstellung selbst oder durch Fremdpflanzen - und welche hergestellt ist aus der Gruppe der nachfolgend aufgelisteten Zielpflanzen, wobei bevorzugte Pflanzenteile der Zielpflanzen in Klammern genannt sind:
- Alchemilla, insbesondere Herba
- Aloysia, insbesondere Herba
- Althaea, insbesondere Radix
- Aspalathus, insbesondere Folium
- Betula, insbesondere Folium
- Borago, insbesondere Folium
- Camellia, insbesondere Folium
- Capsella, insbesondere Herba
- Chinchonae, insbesondere Cortex
- Crataegus, insbesondere Folium c. Flores
- Cynarus, insbesondere Folium
- Echinacea, insbesondere Herba
- Eupatorium, insbesondere Herba
- Euphrasia, insbesondere Herba
- Fumaria, insbesondere Herba
- Ginkgo, insbesondere Folium
- Ginseng, insbesondere Radix
- Hedera, insbesondere Herba
- Herniaria, insbesondere Herba
- Hippocastanus, insbesondere Semen
- Hypericum, insbesondere Herba
- Krameria, insbesondere Radix
- Liquiritia, insbesondere Radix
- Matricaria, insbesondere Folium, Herba
- Melissa, insbesondere Herba
- Mentha, insbesondere Folium
- Orthosiphonis, insbesondere Folium
- Passiflorae, insbesondere Herba
- Pimpinella, insbesondere Fructus
- Pulmonaria, insesondere Herba
- Rosmarinus, insbesondere Folium
- Rubus, insbesondere Folium
- Salicis, insbesondere Cortex
- Senna, insbesondere Folium
- Solidaginis, insbesondere Herba
- Symphytum, insbesondere Herba
- Taraxacum, insbesondere Herba und Radix
- Tussilago, insbesondere Herba
- Thymus, insbesondere Herba
- Tilia, insbesondere Flores
- Vitis vinifera, insbesondere Folium, Fructus

Die im ersten Schritt des erfindungsgemässen Verfahrens oder für die erfindungsgemässe Verwendung von Bentonit eingesetzte pflanzliche Zubereitung muss in flüssigem Zustand vorliegen. Wenn eine Rückverdünnung notwendig ist, beispielsweise von einer lyophilisierten, getrockneten oder hochkonzentrierten pflanzlichen Zubereitung, so soll diese beispielsweise auf einen Trockensubstanzanteil von 0.1% bis 50% erfolgen, insbesondere auf 5% bis 10% Trockensubstanz-Anteil. Die Rückverdünnung oder Verdünnung wird insbesondere mit Wasser, Ethanol oder einem Ethanol/Wasser-Gemisch durchgeführt. Dabei wird eine geeignete Konzentration des Ethanol Wassergemischs bzw. Menge an Ethanol oder Wasser verwendet, so dass in der in Schritt 1 des Verfahrens bereitgestellten flüssigen Zubereitung ein Ethanolgehalt von insbesondere 0.1% m/m bis 50% m/m, bevorzugt ein Ethanolgehalt von 1% m/m bis 10% m/m (Ethanol zu Wasser) erreicht wird. Eine geeignete Ethanolkonzentration berücksichtigt insbesondere die Löslichkeit von erwünschten Wirk- und Leitstoffen und eine abnehmende Entfernung von Schadstoffen durch Absorption an Bentonit beispielsweise in Abhängigkeit einer ansteigenden Ethanolkonzentration in der bereitgestellten flüssigen Zubereitung. Die Grundsubstanz der bereitgestellten flüssigen Zubereitung, respektive das Lösungsmittel für den flüssigen Extrakt oder die Rückverdünnung, wird in den Beispielen auch als ,Matrix` bezeichnet.

Das mineralische Adsorbens Bentonit wird, in insbesondere in Schritt 2 des Verfahrens, mit der flüssigen pflanzlichen Zubereitung in Kontakt gebracht. Vorzugsweise wird Bentonit in die bereitgestellte flüssige pflanzliche Zubereitung eingerührt, gefolgt von einer Einwirkzeit, beispielsweise unter Rühren von 10 Minuten bis zu 4 Stunden, insbesondere eine Einwirkzeit von 30-60 Minuten. Geeignete Temperaturen für das Einrühren und die Einwirkzeit sind insbesondere 5°C bis 60°C, beispielsweise eine Temperatur von 15°C bis 25°C.

Das mineralische Additiv Bentonit wird, insbesondere in Schritt 3 des Verfahrens, bevorzugt durch Filtration, jedoch alternativ auch beispielsweise durch Dekantieren, Zentrifugation oder einer Kombination solcher Abtrennmethoden von der pflanzlichen Zubereitung abgetrennt. Die Abtrennung durch Filtration erfolgt insbesondere durch traditionelle Filtration über Filterschichten, insbesondere Tiefenfilterschichten mit einer Abscheiderate von 50-3 µm, wie beispielsweise die Filterschicht Fibrafix AF-6 der Firma Filtrox AG mit einer Abscheiderate von 35-15 µm, oder beispielsweise durch Filterkerzen, Filterbeutel oder Anschwemmfilter oder via Membrantechnologie, insbesondere Hohlfaser oder Kapillar Röhrenmodule mit einer Porengrösse von 0.1-2.0 µm, wie beispielsweise das Kapillar Modul mit Polypropylen-Membran und einer Porengrösse von 0.2 µm der Firma Mikrodyn-Nadir GmbH.

Die nachfolgenden Beispiele illustrieren die vorliegende Erfindung und dienen der weiteren Erläuterung der Erfindung; sie sollen die Erfindung in keiner Weise limitieren. Die Beispiele 1 bis 23 zeigen verschiedene Ausführungsformen des erfindungsgemässen Verfahrens bzw. der erfindungsgemässen Verwendung von Bentonit. Die fehlende Entfernung von Pyrrolizidin-Alkaloiden durch eine lipophile Phase, wie beispielsweise Glycerol-Monooleat, wird in Vergleichsbeispiel 25 illustriert. Die ungenügende Selektivität für die Entfernung von Pyrrolizidin-Alkaloiden durch andere mineralische Adsorbenzien als Bentonit, wie beispielsweise Aktivkohle oder Bolus alba, wird in den Vergleichsbeispielen 24 und 26 illustriert.

In Schritt 1 der nachfolgenden Beispiele von Ausführungsformen des Verfahrens wurden gemäss den untenstehenden Angaben verschiedene Pflanzenzubereitungen in beispielhaften Varianten bereitgestellt.

In Schritt 2 wurden die bereitgestellten Pflanzenzubereitungen in beispielhaften Varianten gemäss den untenstehenden Angaben mit einem Additiv, meist mit dem mineralischen Adsorbens Bentonit in Kontakt gebracht, bzw. in den Vergleichsbeispielen mit einem anderen genannten Additiv.

In Schritt 3 wurde das Additiv in beispielhaften Varianten gemäss den untenstehenden Angaben abgetrennt.

Anschliessend wurde das resultierende Filtrat konzentriert, wobei die Konzentration beispielhaft unter Vakuum bei einer Temperatur von maximal 50°C auf einen Trockensubstanzgehalt von mindestens 50% erfolgte.

Der Gehalt an Pyrrolizidin-Alkaloiden und an Leit- oder Wirkstoffen in der gereinigten pflanzlichen Zubereitung wie auch in der unbehandelten pflanzlichen Zubereitung vor der Behandlung mit Bentonit ist in den nachfolgenden Tabellen 1 bis 7 aufgeführt. Folgende beispielhafte Analysenmethoden wurden zur Bestimmung des Gehalts an Schadstoffen und Leitstoffen angewendet:
- Pyrrolizidin-Alkaloide (Summe) LC-MS/MS Methode PV 805521 oder 805523, Phytolab GmbH & Co. KG, Vestenbergsgreuth DE
- Apigenin USP
- Rosmarinsäure Ph.Eur 01/2010 :2524
- Flavonoide als Rutin Ph.Eur 01/2013:1874
- Flavonoide als Vitexin Ph.Eur 01/2008:1882
   als HypericinPh.Eur 01/2013:1874

### Beispiel 1

109g Extr. Chamomillae flor. s. spiss. mit einem Trockensubstanzanteil von 55% m/m wurden mit destilliertem Wasser auf einen Trockensubstanzgehalt von 10% m/m rückverdünnt und unter Rühren mit 6g Bentonit versetzt.

Der Extrakt wurde während 30 Minuten bei 40-45°C gerührt und danach direkt via Schichtenfilter filtriert.

### Beispiel 2

200g Extr. Chamomillae flor. s. spiss. mit einem Trockensubstanzanteil von 60% m/m wurden mit destilliertem Wasser auf einen Trockensubstanzgehalt von 5% m/m rückverdünnt und unter Rühren mit 12g Bentonit versetzt.

Der Extrakt wurde während 10 Minuten bei Umgebungstemperatur gerührt und anschliessend auf die Mikrofiltrationsanlage überführt und via Cross-Flow Verfahren filtriert.

Die Polypropylen-Membrane der Mikrofiltrationsanlage wurde vorgängig während 10 Minuten mit destilliertem Wasser bei einer Temperatur von 25-30°C vorkonditioniert.

### Beispiel 3

60g Extr. Chamomillae flor. s. sicc. wurden mit destilliertem Wasser auf einen Trockensubstanzgehalt von 10% m/m rückverdünnt und unter Rühren mit 24g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 4

60g Extr. Chamomillae flor. s. sicc. wurden mit destilliertem Wasser und Ethanol abs. auf einen Trockensubstanzgehalt von 10% m/m und einen Ethanolgehalt von 10% m/m rückverdünnt und unter Rühren mit 24g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 5

60g Extr. Chamomillae flor. a. sicc. wurden mit destilliertem Wasser auf einen Trockensubstanzgehalt von 10% m/m rückverdünnt und unter Rühren mit 24g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 6

60g Extr. Chamomillae flor. a. sicc. wurden mit destilliertem Wasser auf einen Trockensubstanzgehalt von 10% m/m rückverdünnt und unter Rühren mit 42g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 7

60g Extr. Chamomillae flor. a. sicc. wurden mit destilliertem Wasser auf einen Trockensubstanzgehalt von 10% m/m rückverdünnt und unter Rühren mit 60g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 8

30g Extr. Chamomillae flor. a. sicc. wurden mit destilliertem Wasser auf einen Trockensubstanzgehalt von 5% m/m rückverdünnt und unter Rühren mit 3g Bentonit versetzt.

Der Extrakt wurde während 2 Stunden bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 9

60g Extr. Chamomillae flor. a. spiss. mit einem Trockensubstanzanteil von 50% m/m wurden mit destilliertem Wasser auf einen Trockensubstanzgehalt von 5% m/m rückverdünnt und unter Rühren mit 3g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 10

60g Extr. Melissae fol. a. sicc. wurden mit destilliertem Wasser auf einen Trockensubstanzgehalt von 10% m/m rückverdünnt und unter Rühren mit 12g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 11

60g Extr. Melissae fol. a. sicc. wurden mit destilliertem Wasser auf einen Trockensubstanzgehalt von 10% m/m rückverdünnt und unter Rühren mit 24g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 12

100g Extr. Melissae fol. a. sicc. wurden mit destilliertem Wasser auf einen Trockensubstanzgehalt von 10% m/m rückverdünnt und unter Rühren mit 40g Bentonit versetzt.

Der Extrakt wurde während 30 Minuten bei Umgebungstemperatur gerührt und anschliessend auf die Mikrofiltrationsanlage überführt und via Cross-Flow Verfahren filtriert.

Die Polypropylen-Membrane der Mikrofiltrationsanlage wurde vorgängig während 10 Minuten mit destilliertem Wasser bei einer Temperatur von 25-30°C vorkonditioniert.

### Beispiel 13

60g Extr. Melissae fol. a. sicc. wurden mit destilliertem Wasser auf einen Trockensubstanzgehalt von 10% m/m rückverdünnt und unter Rühren mit 42g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 14

60g Extr. Melissae fol. a. sicc. wurden mit destilliertem Wasser auf einen Trockensubstanzgehalt von 10% m/m rückverdünnt und unter Rühren mit 60g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 15

100g Extr. Passiflorae hba. s. spiss. mit einem Trockensubstanzanteil von 60% m/m wurden mit destilliertem Wasser und Ethanol abs. auf einen Trockensubstanzgehalt von 10% m/m und einen Ethanolgehalt von 10% m/m rückverdünnt und unter Rühren mit 12g Bentonit versetzt.

Der Extrakt wurde während 30 Minuten bei Umgebungstemperatur gerührt und anschliessend auf die Mikrofiltrationsanlage überführt und via Cross-Flow Verfahren filtriert.

Die Polypropylen-Membrane der Mikrofiltrationsanlage wurde vorgängig während 10 Minuten mit 10% m/m Ethanol bei einer Temperatur von 25-30°C vorkonditioniert.

### Beispiel 16

100g Extr. Passiflorae hba. s. spiss. mit einem Trockensubstanzanteil von 60% m/m wurden mit destilliertem Wasser auf einen Trockensubstanzgehalt von 10% m/m rückverdünnt und unter Rühren mit 24g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 17

100g Extr. Passiflorae hba. s. spiss. mit einem Trockensubstanzanteil von 60% m/m wurden mit destilliertem Wasser auf einen Trockensubstanzgehalt von 10% m/m rückverdünnt und unter Rühren mit 42g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 18

100g Extr. Passiflorae hba. s. spiss. mit einem Trockensubstanzanteil von 60% m/m wurden mit destilliertem Wasser auf einen Trockensubstanzgehalt von 10% m/m rückverdünnt und unter Rühren mit 60g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 19

60g Extr. Thymi hba. a. sicc. wurden mit destilliertem Wasser auf einen Trockensubstanzgehalt von 10% m/m rückverdünnt und unter Rühren mit 24g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 20

60g Extr. Thymi hba. a. sicc. wurden mit destilliertem Wasser auf einen Trockensubstanzgehalt von 10% m/m rückverdünnt und unter Rühren mit 60g Bentonit versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 21

200g Extr. Hyperici herb..s. spiss. mit einem Trockensubstanzanteil von 60% m/m wurden mit destilliertem Wasser auf einen Trockensubstanzgehalt von 10% m/m rückverdünnt und unter Rühren mit 1.2g geschmolzenem Kokosfett versetzt.

Der Extrakt wurde während 30 Minuten bei einer Temperatur von 40°C gerührt und anschliessend über Nacht bei Umgebungstemperatur stehen gelassen, damit sich ein Bodensatz bilden kann.

Der Überstand wurde abdekantiert und via Schichtenfilter filtriert.

Das Filtrat wurde mit 49g Bentonit versetzt, während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 22

200g Extr. Hyperici herb..s. spiss. mit einem Trockensubstanzanteil von 60% m/m wurden mit destilliertem Wasser auf einen Trockensubstanzgehalt von 10% m/m rückverdünnt und unter Rühren mit 1.2g geschmolzenem Kokosfett versetzt.

Der Extrakt wurde während 30 Minuten bei einer Temperatur von 40°C gerührt und anschliessend über Nacht bei Umgebungstemperatur stehen gelassen, damit sich ein Bodensatz bilden kann.

Der Überstand wurde abdekantiert und via Schichtenfilter filtriert.

Das Filtrat wurde mit 28g Bentonit versetzt, während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 23

200g Extr. Hyperici herb..s. spiss. mit einem Trockensubstanzanteil von 60% m/m wurden mit destilliertem Wasser auf einen Trockensubstanzgehalt von 10% m/m rückverdünnt und unter Rühren mit 1.2g geschmolzenem Kokosfett versetzt.

Der Extrakt wurde während 30 Minuten bei einer Temperatur von 40°C gerührt und anschliessend über Nacht bei Umgebungstemperatur stehen gelassen, damit sich ein Bodensatz bilden kann.

Der Überstand wurde abdekantiert und via Schichtenfilter filtriert.

Das Filtrat wurde mit 70g Bentonit versetzt, während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

### Beispiel 24 (Vergleichsbeispiel)

60g Extr. Chamomillae flor. s. sicc. wurden mit destilliertem Wasser und Ethanol abs. auf einen Trockensubstanzgehalt von 10% m/m und einen Ethanolgehalt von 10% m/m rückverdünnt und unter Rühren mit 6g Aktivkohlepulver versetzt.

Der Extrakt wurde während 2 Stunden bei Umgebungstemperatur gerührt und anschliessend auf die Mikrofiltrationsanlage überführt und via Cross-Flow Verfahren filtriert.

### Beispiel 25 (Vergleichsbeispiel)

60g Extr. Chamomillae flor. a. spiss. wurden mit destilliertem Wasser auf einen Trockensubstanzgehalt von 5% m/m rückverdünnt und unter Rühren mit 3g Glycerol-Monooleat versetzt.

Der Extrakt wurde während 30 Minuten bei Umgebungstemperatur gerührt und anschliessend über Nacht bei 5°C stehengelassen, damit die Fettschicht aushärten kann.

Die Fettschicht an der Oberfläche des Gemisches wurde abgeschöpft und verworfen.

Die verbleibende flüssige Extraktlösung wurde via Schichtenfiltration filtriert.

### Beispiel 26 (Vergleichsbeispiel)

60g Extr. Chamomillae flor. a. sicc. wurden mit destilliertem Wasser auf einen Trockensubstanzgehalt von 5% m/m rückverdünnt und unter Rühren mit 3g Bolus Alba versetzt.

Der Extrakt wurde während 30 Minuten bei Umgebungstemperatur gerührt und danach für 2 Stunden stehen gelassen, damit sich ein Bodensatz bilden kann.

Der Überstand wurde abdekantiert und via Schichtenfilter filtriert.

**Tabelle 1: Beispiele 1-4 (Pflanzenfamilie: Asteraceae, Pflanzengattung: Matricaria)**

| *Additiv Dosierung Matrix Separation* Resultat ^{a)} | Beispiel 1 | | | Beispiel 2 | | | Beispiel 3 | | | Beispiel 4 | | |
|---|---|---|---|---|---|---|---|---|---|---|---|---|
| | *Bentonit 10% m*/*m Wasser Schichtenfiltration* | | | *Bentonit 10% m*/*m Wasser Membranfiltration* | | | *Bentonit 40% m*/*m Wasser Schichtenfiltration* | | | *Bentonit 40% m*/*m 10% m*/*m Ethanol Schichtenfiltration* | | |
| | A | B | A/B | A | B | A/B | A | B | A/B | A | B | A/B |
| PA (Summe) [mg/kg] ^{b)} | 0.409 | 0.187 | 2.2 | 0.409 | 0.19 | 2.2 | 1.411 | 0.157 | 9.0 | 1.232 | 0.132 | 9.3 |

**Tabelle 2a: Beispiele 5-7 (Pflanzenfamilie: Asteraceae, Pflanzengattung: Matricaria)**

| *Additiv Dosierung Matrix Separation* Resultat ^{a)} | Beispiel 5 | | | Beispiel 6 | | | Beispiel 7 | | |
|---|---|---|---|---|---|---|---|---|---|
| | *Bentonit 40% m*/*m Wasser Schichtenfiltration* | | | *Bentonit 70% m*/*m Wasser Schichtenfiltration* | | | *Bentonit 100% m*/*m Wasser Schichtenfiltration* | | |
| | A | B | A/B | A | B | A/B | A | B | A/B |
| PA (Summe) [mg/kg] ^{b)} | 0.349 | 0.136 | 2.6 | 0.349 | 0.083 | 4.2 | 0.349 | 0.074 | 4.7 |

**Tabelle 2b: Beispiele 8-9 (Pflanzenfamilie: Asteraceae, Pflanzengattung: Matricaria)**

| *Additiv Dosierung Matrix Separation* Resultat ^{a)} | Beispiel 8 | | | Beispiel 9 | | |
|---|---|---|---|---|---|---|
| | *Bentonit 10% m*/*m Wasser Schichtenfiltration* | | | *Bentonit 10% m*/*m Wasser Schichtenfiltration* | | |
| | A | B | A/B | A | B | A/B |
| PA (Summe) [mg/kg] ^{b)} | 0.436 | 0.251 | 1.7 | 0.243 | 0.163 | 1.5 |

**Tabelle 3a: Beispiele 10-12 (Pflanzenfamilie: Asteraceae, Pflanzengattung: Melissa)**

| *Additiv Dosierung Matrix Separation* Resultat ^{a)} | Beispiel 10 | | | Beispiel 11 | | | Beispiel 12 | | |
|---|---|---|---|---|---|---|---|---|---|
| | *Bentonit 20% m*/*m* Wasser *Schichtenfiltration* | | | *Bentonit 40% m*/*m* Wasser *Schichtenfiltration* | | | *Bentonit 40*% *m*/*m* Wasser *Membranfiltration* | | |
| | A | B | A/B | A | B | A/B | A | B | A/B |
| PA (Summe) [mg/kg] ^{b)} | 0.847 | 0.113 | 7.5 | 0.874 | 0.037 | 23.6 | 0.847 | 0.107 | 7.9 |
| Rosmarinsäure [% m/m] | 5.36 | 5.26 | 1.0 | 5.36 | 5.42 | 1.0 | 5.36 | 5.67 | 0.9 |

**Tabelle 3b: Beispiele 13-14 (Pflanzenfamilie: Asteraceae, Pflanzengattung: Melissa)**

| *Additiv Dosierung Matrix Separation* Resultat ^{a)} | Beispiel 13 | | | Beispiel 14 | | |
|---|---|---|---|---|---|---|
| | *Bentonit 70% m*/*m* Wasser *Schichtenfiltration* | | | *Bentonit 100% m*/*m* Wasser *Schichtenfiltration* | | |
| | A | B | A/B | A | B | A/B |
| PA (Summe) [mg/kg] ^{b)} | 0.981 | 0.031 | 31.6 | 0.981 | 0.022 | 44.6 |
| Rosmarinsäure [% m/m] | 6.77 | 6.72 | 1.0 | 6.77 | 6.69 | 1.0 |

**Tabelle 4: Beispiele 15-18 (Pflanzenfamilie: Passifloraceae, Pflanzengattung: Passiflora)**

| *Additiv Dosierung Matrix Separation* Resultat ^{a)} | Beispiel 15 | | | Beispiel 16 | | | Beispiel 17 | | | Beispiel 18 | | |
|---|---|---|---|---|---|---|---|---|---|---|---|---|
| | *Bentonit 20% m*/*m 20% m*/*m Ethanol Membranfiltration* | | | *Bentonit 40% m*/*m Wasser Schichtenfiltration* | | | *Bentonit 70% m*/*m Wasser Schichtenfiltration* | | | *Bentonit 100% m*/*m Wasser Schichtenfiltration* | | |
| | A | B | A/B | A | B | A/B | A | B | A/B | A | B | A/B |
| PA (Summe) [mg/kg] ^{b)} | 1.741 | 0.840 | 2.1 | 0.483 | 0.265 | 1.8 | 0.483 | 0.175 | 2.8 | 0.483 | 0.145 | 3.3 |
| Flavonoide als Vitexin [% m/m] | 6.45 | 7.20 | 0.9 | 7.75 | 7.66 | 1.0 | 7.75 | 7. 68 | 1.0 | 7.75 | 7.64 | 1.0 |

**Tabelle 5: Beispiele 19-20 (Pflanzenfamilie: Laminaceae, Pflanzengattung: Thymiane)**

| *Additiv Dosierung Matrix Separation* Resultat ^{a)} | Beispiel 19 | | | Beispiel 20 | | |
|---|---|---|---|---|---|---|
| | *Bentonit 40% m*/*m Wasser Schichtenfiltration* | | | *Bentonit 100% m*/*m Wasser Schichtenfiltration* | | |
| | A | B | A/B | A | B | A/B |
| PA (Summe) [mg/kg] ^{b)} | 0.825 | 0.089 | 9.3 | 0.825 | 0.032 | 25.8 |
| Rosmarinsäure [% m/m] | 3.32 | 3.49 | 1.0 | 3.32 | 3.14 | 1.1 |

**Tabelle 6: Beispiele 21-23 (Pflanzenfamilie: Hypericaceae, Pflanzengattung: Hypericum)**

| *Additiv Dosierung Matrix* Separation Resultat ^{a)} | Beispiel 21 | | | Beispiel 22 | | | Beispiel 23 | | |
|---|---|---|---|---|---|---|---|---|---|
| | *Kokosfett* / *Bentonit 1%* / *40% m*/*m Wasser Schichtenfiltration* | | | *Kokosfett* / *Bentonit 1%* / *70% m*/*m Wasser Schichtenfiltration* | | | *Kokosfett* / *Bentonit 1%* / *100% m*/*m Wasser Schichtenfiltration* | | |
| | A | B | A/B | A | B | A/B | A | B | A/B |
| PA (Summe) [mg/kg] ^{b)} | 0.425 | 0.203 | 2.1 | 0.425 | 0.189 | 2.2 | 0.425 | 0.118 | 3.6 |
| Hypericin [% m/m] | 0.18 | 0.19 | 0.9 | 0.18 | 0.20 | 0.9 | 0.18 | 0.2 | 0.9 |
| Flavonoide als Rutin [% m/m] | 8.12 | 11.20 | 0.7 | 8.12 | 9.40 | 0.9 | 8.12 | 9.21 | 0.9 |

**Tabelle 7: Vergleichsbeispiele 24-26 (Pflanzenfamilie: Asteracea, Pflanzengattung: Matricaria)**

| *Additiv Dosierung Matrix Separation* Resultat ^{a)} | Beispiel 24 | | | Beispiel 25 | | | Beispiel 26 | | |
|---|---|---|---|---|---|---|---|---|---|
| | *Aktivkohle 10% m*/*m 10% m*/*m Ethanol Membranfiltration* | | | *Glycerol-Monooleat 10% m*/*m Wasser Schichtenfiltration* | | | *Bolus Alba 10% m*/*m Wasser Schichtenfiltration* | | |
| | A | B | A/B | A | B | A/B | A | B | A/B |
| PA (Summe) [mg/kg] ^{b)} | 0.368 | 0.177 | 2.1 | 0.306 | 0.303 | 1.0 | 0.37 | 0.377 | 1.0 |
| Apigenin [% m/m] | 3.121 | 1.942 | 1.6 | -- | -- | | -- | -- | |

| | | | | | | | | | |
|---|---|---|---|---|---|---|---|---|---|
| ^{a)} A = Pyrrolizidin-Alkaloid- oder Marker-Gehalt in Ausgangsmaterial = in unbehandelter pflanzliche Zubereitung (Pyrrolizidin-Alkaloid in mg pro kg Ausgangsmaterial; Marker in Massenprozent der Zubereitung pro kg Ausgangsmaterial) B = Pyrrolizidin-Alkaloid- oder Marker-Gehalt in behandeltem Material = in gereinigter pflanzlicher Zubereitung (Pyrrolizidin-Alkaloid in mg pro kg der gereinigten pflanzlichen Zubereitung; Marker in Massenprozent der gereinigten pflanzlichen Zubereitung) A/B = Verhältnis des Pyrrolizidin-Alkaloidgehalts in der gereinigten Zubereitung (A) zur unbehandelten Zubereitung (B) ^{b)} PA (Summe) = Summe aller Pyrrolizidin-Alkaloide (LC-MS/MS Methode PV 805521 oder 805523, Phytolab GmbH & Co. KG, Vestenbergsgreuth DE) | | | | | | | | | |

Alle Analysenresultate beziehen sich auf den Trockensubstanzgehalt der Proben. Analysenresultate, welche die Bestimmungsgrenze unterschritten haben, sind mit einem "kleiner als" Zeichen (<) gekennzeichnet. Entsprechend ist in solchen Fällen das Verhältnis von unbehandelter pflanzlichen Zubereitung zu gereinigter Zubereitung mit einem "grösser als" Zeichen (>) gekennzeichnet

## Patentansprüche

1. Verfahren zur Entfernung von Verunreinigungen umfassend mindestens ein Pyrrolizidin-Alkaloid aus einer pflanzlichen Zubereitung,
wobei das Verfahren folgende Schritte umfasst:
Schritt 1: Bereitstellen einer pflanzlichen Zubereitung in flüssiger Form,
Schritt 2: Kontaktieren der pflanzlichen Zubereitung mit einem Bentonit,
Schritt 3: Abtrennen des Bentonits mit den daran gebundenen Verunreinigungen von der pflanzlichen Zubereitung,
und wobei dadurch eine gereinigte pflanzliche Zubereitung erhalten wird.

2. Verfahren nach Anspruch 1, wobei die Pyrrolizidin-Alkaloide ausgewählt sind aus einer Gruppe umfassend
Pyrrolizidin-Alkaloide des Senecionin-Typs,wie 1,2 ungesättigte Necine, inbesondere Retronecin sowie deren Aminoxide;
Pyrrolizidin-Alkaloide des Lycopsamin-Typs sowie deren Aminoxide; und
Pyrrolizidin-Alkaloide des Triangularin-Typs sowie deren Aminoxide.

3. Verfahren nach einem der vorangehenden Ansprüche zur zusätzlichen Entfernung von mindestens einem lipophilen Schadstoff, wobei
- in einem Schritt A die entsprechende pflanzliche Zubereitung mit einer geeigneten lipophilen Phase in Verbindung gebracht wird und wobei,
- in einem Schritt B die lipophile Phase, mit den darin enthaltenen Schadstoffen von der pflanzlichen Zubereitung abgetrennt wird,
wobei die beiden Schritte A und B vor dem zweiten oder nach dem dritten Schritt durchgeführt werden oder
wobei Schritt A vor, nach oder gleichzeitig mit dem zweiten Schritt und Schritt B vor, nach oder gleichzeitig mit dem dritten Schritt durchgeführt wird.

4. Verfahren nach Anspruch 3, wobei der lipophile Schadstoff ausgewählt ist aus der Gruppe organischer Verbindungen umfassend polyaromatische Kohlenwasserstoffe, insbesondere Benzo[a]anthracen, Chrysen, Benzo[b]fluoranthen, Benzo[a]pyren; polyhalogenierte, insbesondere polychlorierte Biphenyle, Alkylhalogenide und halogenierte Aromate und halogenierte Heteroaromate, insbesondere Chlorpyridine.

5. Verfahren nach einem der vorangehenden Ansprüche, wobei die flüssige pflanzliche Zubereitung ausgewählt ist aus der Gruppe umfassend Aufgüsse, Tinkturen, Fluida, Spissum-Extrakte, Tropflösungen, Decocte, Mazerate, Digestione und Tonika sowie flüssige Zwischenprodukte von getrockneten Zubereitungen oder flüssige Zubereitungen, die aus Konzentraten oder aus getrockneten Zubereitungen hergestellt werden.

6. Verfahren nach einem der vorangehenden Ansprüche, wobei die bereitgestellte pflanzliche Zubereitung aus einer Pyrrolizidin Alkaloid führenden Pflanze oder Pflanzenteilen hergestellt wird und wobei die genannten Pflanzen oder die genannten Pflanzenteile mindestens einen erwünschten Inhaltsstoff enthalten.

7. Verfahren nach einem der vorangehenden Ansprüche, wobei der mindestens eine erwünschte Inhaltsstoff insbesondere ausgewählt ist aus der Gruppe umfassend Polyphenole, insbesondere Rosmarinsäure oder Flavonoide und Flavone wie Rutin, Apigenin oder Vitexin, Hypericine, wie Hypericin, Saponine, Isoprenoide und Iridoide, ätherische Öle, Alkylamide, Pflanzensäuren, wie zum Beispiel, Cichoriensäure, Lignane, Anthocyane, Anthracen-Derivate, Terpene, beispielsweise Sesquiterpenlactone oder Polyterpene, Cumarine, Glukosinolate, Bitterstoffe und Allantoin.

8. Verfahren nach einem der vorangehenden Ansprüche, wobei die bereit gestellte pflanzliche Zubereitung hergestellt ist aus Pflanzen oder insbesondere Pflanzenteilen ausgewählt aus der Gruppe umfassend
- Alchemilla, insbesondere Herba
- Aloysia, insbesondere Herba
- Althaea, insbesondere Radix
- Aspalathus, insbesondere Folium
- Betula, insbesondere Folium
- Borago, insbesondere Folium
- Camellia, insbesondere Folium
- Capsella, insbesondere Herba
- Chinchonae, insbesondere Cortex
- Crataegus, insbesondere Folium c. Flores
- Cynarus, insbesondere Folium
- Echinacea, insbesondere Herba
- Eupatorium, insbesondere Herba
- Euphrasia, insbesondere Herba
- Fumaria, insbesondere Herba
- Ginkgo, insbesondere Folium
- Ginseng, insbesondere Radix
- Hedera, insbesondere Herba
- Herniaria, insbesondere Herba
- Hippocastanum, insbesondere Semen
- Hypericum, insbesondere Herba
- Krameria, insbesondere Radix
- Liquiritia, insbesondere Radix
- Matricaria, insbesondere Folium, Herba
- Melissa, insbesondere Herba
- Mentha, insbesondere Folium
- Orthosiphonis, insbesondere Folium
- Passiflorae, insbesondereHerba
- Pimpinella, insbesondere Fructus
- Pulmonaria, insesondere Herba
- Rosmarinus, insbesondere Folium
- Rubus, insbesondere Folium
- Salicis, insbesondere Cortex
- Senna, insbesondere Folium
- Solidaginis, insbesondere Herba
- Symphytum, insbesondere Herba
- Taraxacum, insbesondere Herba und Radix
- Tussilago, insbesondere Herba
- Thymus, insbesondere Herba
- Tilia, insbesondere Flores
- Vitis vinifera, insbesondere Folium, Fructus

9. Verfahren nach einem der vorangehenden Ansprüche, wobei Bentonit in einer Menge von 5 bis 200% m/m, bezogen auf den Trockensubstanzgehalt der Zubereitung zugegeben wird,
wobei eine Mindestmenge an zugegebenem Bentonit insbesondere ausgewählt wird aus mindestens 10% m/m, 20% m/m, 30%, 40% m/m, 50% m/m, 70% m/m oder 100% m/m und/oder
wobei eine maximale Menge an zugegebenem Bentonit insbesondere ausgewählt wird aus bis maximal 30% m/m, 40% m/m, 50% m/m 70 % m/m oder bis 100% m/m.

10. Verwendung von Bentonit zur Entfernung von Verunreinigungen umfassend mindestens ein Pyrrolizidin-Alkaloid aus einer pflanzlichen Zubereitung, wobei Bentonit insbesondere in einem Verfahren gemäss einem der Ansprüche 1 bis 9 verwendet wird.

## Claims

1. Method for removing impurities comprising at least one pyrrolizidine alkaloid from a plant preparation,
said method comprising the following steps:
Step 1: providing a plant preparation in liquid form,
Step 2: contacting the plant preparation with a bentonite,
Step 3: separating the bentonite with the impurities bound thereto from the herbal preparation,
and thereby obtaining a purified plant preparation.

2. Method according to claim 1, wherein the pyrrolizidine alkaloids are selected from a group comprising
pyrrolizidine alkaloids of the senecionine type, such as 1,2 unsaturated necines, in particular retronecine, and their amine oxides;
pyrrolizidine alkaloids of the lycopsamine type and their amine oxides; and
Pyrrolizidine alkaloids of the triangularine type as well as their amine oxides.

3. Method according to one of the preceding claims for the additional removal of at least one lipophilic pollutant, wherein
- in a step A the corresponding plant preparation is brought into contact with a suitable lipophilic phase and wherein,
- in a step B, the lipophilic phase, together with the pollutants contained therein, is separated from the plant preparation,
wherein both steps A and B are carried out before the second or after the third step, or
wherein step A is carried out before, after or simultaneously with the second step and step B is carried out before, after or simultaneously with the third step.

4. Method according to claim 3, wherein the lipophilic pollutant is selected from the group of organic compounds comprising polyaromatic hydrocarbons, in particular benzo[a]anthracene, chrysene, benzo[b]fluoranthene, benzo[a]pyrene; polyhalogenated, in particular polychlorinated biphenyls, alkyl halides and halogenated aromatics and halogenated heteroaromatics, in particular chloropyridines.

5. Method according to one of the preceding claims, wherein the liquid plant preparation is selected from the group comprising infusions, tinctures, fluids, spissum extracts, drip solutions, decoctes, macerates, digestiones and tonics, as well as liquid intermediates of dried preparations or liquid preparations prepared from concentrates or from dried preparations.

6. Method according to one of the preceding claims, wherein the prepared plant preparation is prepared from a pyrrolizidine alkaloid-bearing plant or plant parts, and wherein said plants or said plant parts contain at least one desired ingredient.

7. Method according to one of the preceding claims, wherein said at least one desired ingredient is in particular selected from the group comprising polyphenols, in particular rosmarinic acid or flavonoids and flavones such as rutin, apigenin or vitexin, hypericins, such as hypericin, saponins, isoprenoids and iridoids, essential oils, alkylamides, plant acids, such as, for example, cichoric acid, lignans, anthocyanins, anthracene derivatives, terpenes, for example, sesquiterpene lactones or polyterpenes, coumarins, glucosinolates, bitter substances and allantoin.

8. Method according to one of the preceding claims, wherein the provided plant preparation is prepared from plants or in particular plant parts selected from the group comprising
- Alchemilla, in particular Herba
- Aloysia, in particular Herba
- Althaea, in particular Radix
- Aspalathus, in particular Folium
- Betula, in particular Folium
- Borago, in particular Folium
- Camellia, in particular Folium
- Capsella, in particular Herba
- Chinchonae, in particular Cortex
- Crataegus, in particular Folium c. Flores
- Cynarus, in particular Folium
- Echinacea, in particular Herba
- Eupatorium, in particular Herba
- Euphrasia, in particular Herba
- Fumaria, in particular Herba
- Ginkgo, in particular Folium
- Ginseng, in particular Radix
- Hedera, in particular Herba
- Herniaria, in particular Herba
- Hippocastanum, in particular Semen
- Hypericum, in particular Herba
- Krameria, in particular Radix
- Liquiritia, in particular Radix
- Matricaria, in particular Folium, Herba
- Melissa, in particular Herba
- Mentha, in particular Folium
- Orthosiphonis, in particular Folium
- Passiflorae, in particularHerba
- Pimpinella, in particular Fructus
- Pulmonaria, in particular Herba
- Rosmarinus, in particular Folium
- Rubus, in particular Folium
- Salicis, in particular Cortex
- Senna, in particular Folium
- Solidaginis, in particular Herba
- Symphytum, in particular Herba
- Taraxacum, in particular Herba and Radix
- Tussilago, in particular Herba
- Thymus, in particular Herba
- Tilia, in particular Flores
- Vitis vinifera, in particular Folium, Fructus.

9. Method according to one of the preceding claims, wherein bentonite is added in an amount of 5 to 200% m/m, based on the dry substance content of the preparation,
wherein a minimum amount of added bentonite is in particular selected from at least 10% m/m, 20% m/m, 30%, 40% m/m, 50% m/m, 70% m/m or 100% m/m and/or
wherein a maximum amount of added bentonite is in particular selected from up to a maximum of 30% m/m, 40% m/m, 50% m/m 70% m/m or up to 100% m/m.

10. Use of bentonite for removing impurities comprising at least one pyrrolizidine alkaloid from a plant preparation, wherein bentonite is used in particular in a method according to one of the claims 1 to 9.

## Revendications

1. Procédé d'élimination des impuretés comprenant au moins un alcaloïde pyrrolizidinique dans une préparation végétale,
le procédé comprenant les étapes suivantes :
Étape 1 : fournir une préparation à base de plantes sous forme liquide,
Étape 2 : mettre en contact la préparation à base de plantes avec une bentonite,
Étape 3 : séparer la bentonite avec les impuretés qui y sont liées de la préparation à base de plantes,
et obtenir ainsi une préparation à base de plantes purifiée.

2. Procédé selon la revendication 1, dans lequel les alcaloïdes pyrrolizidiniques sont choisis dans un groupe comprenant
des alcaloïdes pyrrolizidiniques du type senecionine, tels que les nécines 1,2 insaturées, en particulier la rétronécine, ainsi que leurs oxydes d'amine ;
des alcaloïdes pyrrolizidiniques du type lycopsamine et leurs oxydes d'amine ; et
des alcaloïdes pyrrolizidiniques de type triangulairine et leurs oxydes d'amine.

3. Procédé selon l'une des revendications précédentes pour l'élimination supplémentaire d'au moins un polluant lipophile, dans lequel
- dans une étape A, la préparation à base de plantes correspondante est mise en contact avec une phase lipophile appropriée et dans lequel,
- dans une étape B, la phase lipophile avec les polluants qu'elle contient, sont séparés de la préparation à base de plantes,
dans lequel les deux étapes A et B sont effectuées avant la deuxième ou après la troisième étape, ou
dans lequel l'étape A est effectuée avant, après ou simultanément à la deuxième étape et l'étape B est effectuée avant, après ou simultanément à la troisième étape.

4. Procédé selon la revendication 3, dans lequel le polluant lipophile est choisi dans le groupe des composés organiques comprenant les hydrocarbures polyaromatiques, en particulier le benzo[a]anthracène, le chrysène, le benzo[b]fluoranthène, le benzo[a]pyrène ; les polyhalogénés, en particulier les polychlorobiphényles, les halogénures d'alkyle et les aromatiques halogénés et les hétéroaromatiques halogénés, en particulier les chloropyridines.

5. Procédé selon l'une des revendications précédentes, dans lequel la préparation à base de plantes liquide est choisie dans le groupe comprenant les infusions, les tinctures, les fluides, les extraits de spissum, les solutions de goutte à goutte, les décoctés, les macérats, les digestifs et les toniques, ainsi que les intermédiaires liquides de préparations séchées ou les préparations liquides préparées à partir de concentrés ou de préparations séchées.

6. Procédé selon l'une des revendications précédentes, dans lequel la préparation à base de plantes fournie est préparée à partir d'une plante ou de parties de plante contenant des alcaloïdes de la pyrrolizidine, et dans lequel ces plantes ou ces parties de plante contiennent au moins un ingrédient souhaité.

7. Procédé selon l'une des revendications précédentes, dans lequel ledit au moins un ingrédient souhaité est notamment choisi dans le groupe comprenant les polyphénols, en particulier l'acide rosmarinique ou les flavonoïdes et flavones tels que la rutine, l'apigénine ou la vitexine, les hypéricines, telles que l'hypéricine, saponines, isoprénoïdes et iridoïdes, huiles essentielles, alkylamides, acides végétaux, tels que, par exemple, l'acide cichorique, lignanes, anthocyanes, dérivés anthracéniques, terpènes, par exemple, lactones sesquiterpéniques ou polyterpènes, coumarines, glucosinolates, substances amères et allantoïne.

8. Procédé selon l'une des revendications précédentes, dans lequel la préparation à base de plantes fournie est préparée à partir de plantes ou en particulier de parties de plantes choisies dans le groupe comprenant
- Alchemille, en particulier Herba
- Aloysia, en particulier Herba
- Althaea, en particulier Radix
- Aspalathus, en particulier Folium
- Betula, en particulier Folium
- Borago, en particulier Folium
- Camélia, en particulier Folium
- Capsella, en particulier Herba
- Chinchonae, en particulier Cortex
- Crataegus, en particulier Folium c. Flores
- Cynarus, en particulier Folium
- Echinacea, en particulier Herba
- Eupatorium, en particulier Herba
- Euphrasie, en particulier Herba
- Fumaria, en particulier Herba
- Ginkgo, en particulier Folium
- Ginseng, en particulier Radix
- Hedera, en particulier Herba
- Herniaria, en particulier Herba
- Hippocastanum, en particulier Semen
- Hypericum, en particulier Herba
- Krameria, en particulier Radix
- Liquiritia, en particulier Radix
- Matricaria, en particulier Folium, Herba
- Melissa, en particulier Herba
- Mentha, en particulier Folium
- Orthosiphonis, en particulier Folium
- Passiflorae, en particulier Herba
- Pimpinella, en particulier Fructus
- Pulmonaria, en particulier Herba
- Rosmarinus, en particulier Folium
- Rubus, en particulier Folium
- Salicis, en particulier Cortex
- Séné, en particulier Folium
- Solidaginis, en particulier Herba
- Symphytum, en particulier Herba
- Taraxacum, en particulier Herba et Radix
- Tussilago, en particulier Herba
- Thymus, en particulier Herba
- Tilia, en particulier Flores
- Vitis vinifera, en particulier Folium, Fructus.

9. Procédé selon l'une des revendications précédentes, dans lequel de la bentonite est ajoutée dans une proportion de 5 à 200% m/m, par rapport à la teneur en matière sèche de la préparation,
dans lequel la quantité minimale de bentonite ajoutée est notamment choisie parmi au moins 10% m/m, 20% m/m, 30%, 40% m/m, 50% m/m, 70% m/m ou 100% m/m et/ou
dans lequel la quantité maximale de bentonite ajoutée est notamment choisie parmi un maximum de 30% m/m, 40% m/m, 50% m/m, 70% m/m ou jusqu'à 100% m/m.

10. Utilisation de la bentonite pour éliminer les impuretés comprenant au moins un alcaloïde pyrrolizidinique d'une préparation à base de plantes, la bentonite étant utilisée en particulier dans un procédé selon l'une des revendications 1 à 9.
